# EUROPÄISCHE PATENTSCHRIFT

(11) **EP 2 461 846 B1**
(45) Veröffentlichungstag und Bekanntmachung des Hinweises auf die Patenterteilung: **05.09.2018**
(21) Anmeldenummer: 10742732.0
(22) Anmeldetag: 29.07.2010
(51) Int. Cl.: A61M 1/14, A61M 1/36

(54) **VERBINDUNGSVORRICHTUNG ZUM VERBINDEN WENIGSTENS EINER EXTERNEN FUNKTIONSEINRICHTUNG MIT EINER ANORDNUNG SOWIE ANORDNUNG UMFASSEND EINE SOLCHE VERBINDUNGSVORRICHTUNG**
CONNECTING DEVICE FOR CONNECTING AT LEAST ONE EXTERNAL FUNCTIONAL APPARATUS TO AN ARRANGEMENT, AND ARRANGEMENT COMPRISING A CONNECTING DEVICE OF SAID KIND
DISPOSITIF DE LIAISON POUR RELIER AU MOINS UN DISPOSITIF DE FONCTION EXTERNE AVEC UN AGENCEMENT, ET AGENCEMENT COMPRENANT UN TEL DISPOSITIF DE LIAISON

(30) Priorität: 04.08.2009 DE 102009036101
(43) Veröffentlichungstag der Anmeldung: 13.06.2012
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2010/004642
(87) Internationale Veröffentlichungsnummer: WO 2011/015309

(56) Entgegenhaltungen:
- WO-A1-2009/051669
- DE-A1- 4 419 593
- US-A1- 2005 020 959

## Beschreibung

Die vorliegende Erfindung betrifft eine Verbindungsvorrichtung zum Verbinden wenigstens einer externen Funktionseinrichtung mit einer Anordnung, welche eine Blutbehandlungsvorrichtung ist, mittels Verpressen der externen Funktionseinrichtung gemäß dem Oberbegriff des Anspruch 1. Die vorliegende Erfindung betrifft zudem eine Anordnung gemäß dem Anspruch 9, welche eine erfindungsgemäße Verbindungsvorrichtung umfasst.

Bei Anordnungen, wie beispielsweise medizintechnischen Behandlungsvorrichtungen, labortechnischen Anordnungen oder auch Anordnungen zur Nahrungsmittelherstellung, ist oftmals ein Verbinden, insbesondere Ankoppeln, externer Funktionseinrichtungen, wie Schläuche, Wärmetauscher, Messkammern oder multifunktionale Disposablekassetten, an der Anordnung vor deren Verwendung erforderlich.

Das Verbinden oder Ankoppeln einer solchen externen Funktionseinrichtung an die Anordnung erfolgt mittels einer Verbindungsvorrichtung mit einer Aufnahmeeinrichtung zum Aufnehmen der externen Funktionseinrichtung und einer Verpresseinrichtung zum Ausüben von Druck auf die externe Funktionseinrichtung.

Aus der DE 44 19 593 A1 ist eine Vorrichtung zum Messen des Drucks eines Mediums bekannt.

Aus der US 2005/020959 A1 ist eine modulare medizinische, ersetzbare Komponente bekannt.

Aufgabe der vorliegenden Erfindung ist, eine weitere Verbindungsvorrichtung zum Verbinden wenigstens einer Funktionseinrichtung mit einer Anordnung bereitzustellen. Ferner ist es Ziel der vorliegenden Erfindung, eine Anordnung, welche eine derartige Verbindungsvorrichtung aufweist, anzugeben.

Die erfindungsgemäße Aufgabe wird durch eine Verbindungsvorrichtung mit den Merkmalen des Anspruchs 1 gelöst.

Die erfindungsgemäße Verbindungsvorrichtung weist wenigstens eine Aufnahmeeinrichtung mit wenigstens einem ersten Kontaktabschnitt zum Aufnehmen wenigstens einer externen Funktionseinrichtung zwischen dem ersten Kontaktabschnitt und einem oder mehreren weiteren Kontaktabschnitten auf. Sie weist ferner wenigstens eine Verpresseinrichtung zum Verpressen der wenigstens einen externen Funktionseinrichtung zwischen dem ersten Kontaktabschnitt und dem weiteren Kontaktabschnitt auf. Die Verpresseinrichtung ist ausgestaltet, um wenigstens den ersten Kontaktabschnitt unter Aufbringung einer ersten Federkraft F₁ und einer größeren zweiten Federkraft F₂ von einer ersten Stellung, insbesondere einer Rüststellung, in eine zweite Stellung zu überführen.

Eine "externe Funktionseinrichtung", wie sie hierin verwendet wird, kann ein Schlauch, ein Wärmetauscher, eine Messeinrichtung, eine multifunktionale Disposablekassette, ein beliebiger Einmalartikel oder dergleichen sein. Dabei gilt im Zusammenhang mit der vorliegenden Offenbarung, dass der Begriff "kann aufweisen" bzw. "kann sein" synonym zum hier und auch an anderer Stelle ebenfalls verwendeten Begriff "weist bevorzugt auf" bzw. "ist bevorzugt" ist.

Die externe Funktionseinrichtung kann Energie, Messwerte und/oder mechanische Bewegungen und Kräfte auf die Anordnung übertragen oder von dieser empfangen. Sie kann jedoch von der Anordnung auch nur gehalten werden und mit dieser nicht in Wechselwirkung oder Signalkommunikation stehen.

Eine "Anordnung" im Sinne der vorliegenden Erfindung kann eine medizintechnische Anordnung, wie beispielsweise eine Blutbehandlungsvorrichtung, z.B. eine Dialysiervorrichtung, eine Anordnung in der Labortechnik, eine Anordnung in der Arznei- oder Lebensmittelherstellung oder dergleichen, sein.

Der Begriff "Verpressen", wie er hierin verwendet wird, kann erfindungsgemäß ein Halten mittels Druck, insbesondere mittels Pressen, Quetschen oder dergleichen bedeuten.

Die erfindungsgemäße Verbindungsvorrichtung kann zum Ankoppeln einer oder mehrerer externer Funktionseinrichtungen vorgesehen sein.

Das Pressen oder Verpressen kann bevorzugt vorübergehender Natur sein. Die mittels Verpressen angekoppelte externe Funktionseinrichtung kann lösbar befestigt sein. Sie kann austauschbar befestigt sein.

Es können neben der Aufnahmeeinrichtung weitere Einrichtungen zum Verpressen bzw. Halten der externen Funktionseinrichtung, wie beispielsweise Schlaufen, Ösen, Spanngurte, Schnappverschlüsse, Hebel und dergleichen, vorgesehen sein.

Der Begriff "Verbinden" und/oder "Ankoppeln", wie er hierin verwendet wird, kann ein funktionelles und/oder mechanisches Verbinden der externen Funktionseinrichtung mit einem Koppelpartner auf der Seite der Anordnung bezeichnen oder umfassen.

Ein "Koppelpartner der Anordnung" kann z.B. eine Messeinrichtung, wie beispielsweise ein Sensor, sein.

Eine "Aufnahmeeinrichtung" im Sinne der vorliegenden Erfindung ist eine Einrichtung, die zum Aufnehmen wenigstens einer externen Funktionseinrichtung geeignet ist.

Die Abmessung einer Öffnung oder eines Spalts der Aufnahmeeinrichtung zum Aufnahmen der externen Funktionseinrichtung oder der externen Funktionseinrichtungen kann entsprechend der Höhe, der Länge, der Breite, dem Durchmesser oder dergleichen der externen Funktionseinrichtung oder der externen Funktionseinrichtungen zu deren konkreter Aufnahme ausgestaltet sein.

Die Abmessung kann veränderbar ausgestaltet sein.

Die Aufnahmeeinrichtung weist wenigstens einen ersten Kontaktabschnitt auf.

Der "erste Kontaktabschnitt" kann eine Andruckplatte sein. Er kann flach oder gekrümmt ausgestaltet sein. Er kann punktförmig oder flächig mit der externen Funktionseinrichtung in Kontakt treten. Er kann einen Abschnitt aufweisen, welcher in seiner Geometrie einer Erstreckung der anzukoppelnden externen Funktionseinrichtung - im verpressten oder im nicht verpressten Zustand - entspricht.

Der erste Kontaktabschnitt kann multifunktionell zur Grobausrichtung, zur Verrastung und/oder zur Übertragung einer Verpresskraft auf die externe Funktionseinrichtung dienen.

Der erste Kontaktabschnitt kann gegebenenfalls weitere Einrichtungen zum Unterstützen eines Verpressens der externen Funktionseinrichtung und/oder Halten derselben aufweisen.

Der "weitere Kontaktabschnitt" ist bevorzugt an der erfindungsgemäßen Verbindungsvorrichtung selbst oder an einer Anordnung vorgesehen.

Der weitere Kontaktabschnitt kann wie der erste Kontaktabschnitt ausgestaltet sein. Er kann eine Ankoppelfläche sein. Der Begriff "Ankoppelfläche" kann beispielsweise wenigstens einen Abschnitt einer Oberseite eines Trägerelements und/oder eines Stützelements und/oder einer Messeinrichtung oder dergleichen der Anordnung oder der Verbindungsvorrichtung bezeichnen.

Der erste Kontaktabschnitt ist beweglich ausgestaltet. Der weitere Kontaktabschnitt kann beweglich ausgestaltet sein. Wenigstens einer der Kontaktabschnitte kann starr angeordnet sein.

Der erste Kontaktabschnitt ist mit einem variierbaren Abstand d zum weiteren Kontaktabschnitt angeordnet. Der Abstand d kann insbesondere variiert werden, indem der erste Kontaktabschnitt in Richtung auf den weiteren Kontaktabschnitt hin bewegt wird.

Die Kontaktabschnitte können zusammen oder mit wenigstens einem weiteren Abschnitt der erfindungsgemäßen Verbindungsvorrichtung oder einer Anordnung, wie beispielsweise einem Trägerelement, einen C-förmigen Abschnitt bilden.

Die Aufnahmeeinrichtung kann wenigstens einen in seiner Höhe oder Breite variierbaren Einbauspalt aufweisen, in den die externe Funktionseinrichtung in der Rüststellung einlegbar ist und aus dem die externe Funktionseinrichtung bei Bedarf wieder entnehmbar ist.

Eine "Verpresseinrichtung" im Sinne der vorliegenden Erfindung ist zum Verpressen der externen Funktionseinrichtung durch Überführen wenigstens des ersten Kontaktabschnitts und/oder des weiteren Kontaktabschnitts aus einer ersten Stellung der Verbindungsvorrichtung in eine zweite Stellung, in welcher mittels der Kontaktabschnitte Druck auf die externe Funktionseinrichtung ausgeübt wird, ausgelegt.

Das "Verpressen" kann durch Aufbringen einer ersten und einer zweiten Federkraft F₁ bzw. F₂ auf wenigstens den ersten Kontaktabschnitt erreicht werden.

Die erste Stellung kann eine Rüststellung sein.

Eine "Rüststellung" im Sinne der vorliegenden Erfindung ist eine Stellung, bei welcher der erste Kontaktabschnitt mit einem Abstand d₁ vom weiteren Kontaktabschnitt beabstandet ist.

In der Rüststellung ist die Öffnung der Aufnahmeeinrichtung groß genug, um das Einlegen wenigstens einer externen Funktionseinrichtung zuzulassen.

Ferner kann die Öffnung auch so groß sein, dass Körperteile, insbesondere Finger, in diesen einführbar sind.

Die "zweite Stellung" ist erfindungsgemäß eine Stellung, in welcher der erste Kontaktabschnitt vom weiteren Kontaktabschnitt mit einem Abstand d₂ beabstandet ist, wobei der zweite Abstand d₂ kleiner als der erste Abstand d₁ ist.

In der zweiten Stellung kann die Öffnung der Verbindungsvorrichtung zu klein sein zum Einlegen der externen Funktionseinrichtung und/oder zum Einführen von Körperteilen.

Aufgrund des Aufbringens zweier unterschiedlicher Federkräfte F₁ und F₂ beim Betätigen der Verpresseinrichtung ist erfindungsgemäß eine gestufte oder sprungweise Kraftwirkung erzielbar. Eine derartige gestufte Kraftwirkung kann einen gezielten Einfluss auf Parameter der Bewegung, wie beispielsweise die Geschwindigkeit, die Zeitdauer der Bewegung, die Beschleunigung, die Kraftverteilung, Stellungen und dergleichen ermöglichen. Sie kann ferner einen variabel gestaltbaren Bauraum der Verbindungsvorrichtung, der Anordnung, von einzelnen Komponenten derselben und/oder weiteren Einrichtungen und Elementen zulassen.

Vorteilhafte Weiterentwicklungen der vorliegenden Erfindung sind jeweils Gegenstand der Unteransprüche.

Erfindungsgemäß ist die Verpresseinrichtung ausgestaltet, um wenigstens den ersten Kontaktabschnitt in Abhängigkeit des Abstandes d mit der ersten Federkraft F₁ und/oder der zweiten Federkraft F₂ von der ersten Stellung in die zweite Stellung zu überführen.

Die zum Überführen des wenigstens einen beweglichen Kontaktabschnitts zwischen der ersten und der zweiten Stellung erforderliche Federkraft kann in Abhängigkeit des aktuellen, also tatsächlichen, Abstands d zwischen dem ersten Kontaktabschnitt und dem weiteren Kontaktabschnitt bemessen oder variabel sein.

Der Übergang von der ersten Stellung in die zweite Stellung kann gezielt, d.h. auf vorher festgelegte oder vorbestimmte Weise erfolgen. Insbesondere können Sprünge im Kraftverlauf vorgesehen sein.

Erfindungsgemäß weist die Verpresseinrichtung wenigstens zwei Federn zum Überführen wenigstens des ersten Kontaktabschnitts aus der ersten Stellung in die zweite Stellung auf.

Die Federn können zum Aufnehmen und Speichern von Energie und/oder Übertragen von Kraft geeignet sein.

Die Verpresseinrichtung kann in einer nicht-erfindungsgemäßen Ausführungsform vorgesehen nur eine einzige Feder aufweisen, wobei diese einen nicht stetigen oder "geknickten" wegabhängigen Kraftverlauf in einem Kraft-Weg-Diagramm beim Übergang von einer Stellung in einen andere aufweist oder erzeugt. Hierzu ggf. erforderliche zusätzliche Anschläge und Koppelglieder können von der Verpresseinrichtung ferner umfasst sein. In einer bevorzugten Ausführungsform der vorliegenden Erfindung ist die erste Feder ausgestaltet, um eine erste Federkraft F₁ aufzubringen und/oder zu übertragen und eine zweite Feder ist ausgestaltet, um eine zweite Federkraft F₂ aufzubringen und/oder zu übertragen. Die erste Federkraft F₁ ist kleiner bzw. schwächer als die zweite Federkraft F₂.

Die erste Federkraft F₁ und/oder die zweite Federkraft F₂ können jeweils einen Kraftbereich oder Kräftebereich bezeichnen bzw. einen solchen ausmachen. Sie können andererseits jedoch auch ein diskretes Kraftniveau bzw. eine absolute Kraft bezeichnen. Die erste Federkraft F₁ und die zweite Federkraft F₂ können einander auch überlappen, d.h. die Kraftbereiche derselben können ineinander übergehen.

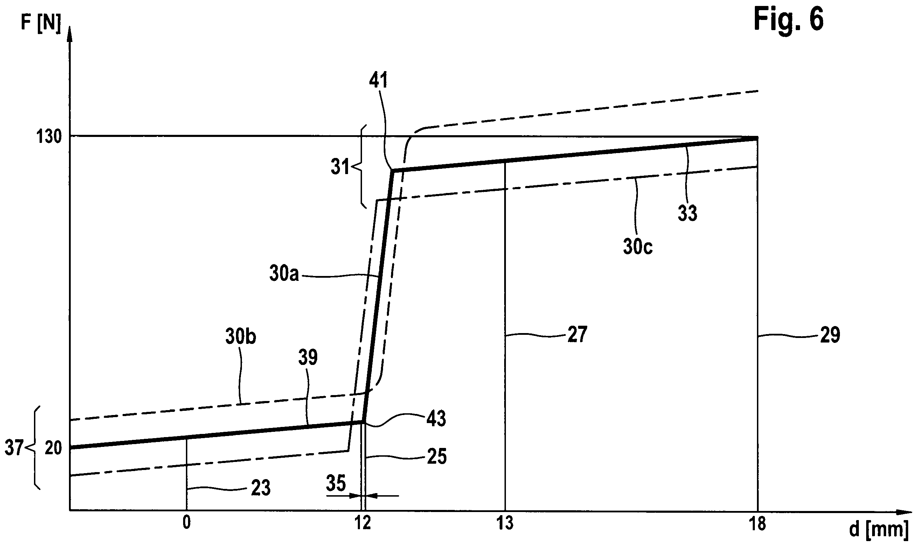

Beispiele für Kraftbereiche der Federkräfte F₁ und F₂ sind in Fig. 6 gezeigt. So kann F₁ z. B. einen Kraftbereich geringerer Kräfte und F₂ einen Kraftbereich höherer Kräfte darstellen oder bezeichnen.

In einer noch weiter bevorzugten Ausführungsform der vorliegenden Erfindung sind die erste und die zweite Feder derart angeordnet, dass die erste Feder während des Übergangs von der ersten Stellung zu einer hier als Kraftwechselstellung bezeichneten Stellung die erste Federkraft F₁ aufbringt und/oder überträgt und die zweite Feder während des Übergangs von der Kraftwechselstellung zur zweiten Stellung die zweite Federkraft F₂ aufbringt und/oder überträgt.

Während des Übergangs von der ersten Stellung in die zweite Stellung wird zunächst die erste Federkraft F₁ aufgebracht. Nach Erreichen der Kraftwechselstellung wird die zweite Federkraft F₂ aufgebracht. Die erste Federkraft F₁ kann eine Summe von Kräften unter Vernachlässigung von Reibungs- und anderen Kräften sein. Die zweite Federkraft F₂ kann exakt oder annähernd der Kraft FE₂ der zweiten Feder entsprechen.

Da Überschneidungen möglich sind, ist die Kraftwechselstellung nicht ausschließlich als definierter Punkt zu verstehen, an dem der Kraftwechsel stattfindet. Vielmehr kann die Kraftwechselstellung einen Übergangsbereich mit wesentlicher Änderung der auf die Kontaktabschnitte wirkenden Kraft bezeichnen.

Die erste Federkraft F₁ ist kleiner bzw. schwächer als die zweite Federkraft F_{2,} wie beispielsweise im Kraft-Weg-Diagramm der Fig. 6 gezeigt ist.

Die erste Federkraft F₁ kann eine größere Verfahrgeschwindigkeit v des bewegten Kontaktabschnitts als die zweite Federkraft F₂ bewirken. Die Geschwindigkeit, mit der die erfindungsgemäße Verbindungsvorrichtung beim Übergang von der ersten Stellung in die zweite Stellung verfahren wird, kann an der Kraftwechselstellung von einer höheren Geschwindigkeit in eine niedrigere Geschwindigkeit übergehen.

In einer weiter bevorzugten Ausführungsform der vorliegenden Erfindung sind die Federn als Spannungs-Dehnungs-Elemente ausgestaltet.

Die erste und die zweite Feder können lösbar oder unlösbar mit Federdübeln, wie sie exemplarisch in der beigefügten Zeichnung und ihrer Beschreibung erläutert sind, und/oder Bewegungsanschlägen kombiniert angeordnet sein.

Die erste und die zweite Feder können parallel geschaltet und/oder - vorzugsweise koaxial - ineinander geschachtelt sein. Sie können seriell geschaltet, parallel nebeneinander angeordnet oder koaxial hintereinander angeordnet sein.

Die erste und die zweite Feder können Schraubenfedern, gewundene Torsionsfedern, Schenkelfedern, Torsions- und/oder Drehstabfedern, Biegefedern, Spiralfedern, Blattfedern, Tellerfedern, Membranfedern, Luftfedern, Gasdruckfedern, Elastomerfedern, Ringfedern oder dergleichen sein.

Die Federn können aus Federstählen, wie z.B. 38Si7, 61SiCr7, 52CrMoV4, 51CrV4 (nach EN 10089), Federstahl 1.4310, aus Kupfer-Beryllium-Legierungen, Gummi, Faserverbundwerkstoffen, wie glasfaserverstärkten Kunststoffen aus Polyesterharz, Epoxidharz oder Polyamid, Nickellegierungen und/oder dergleichen hergestellt sein oder solche aufweisen.

In einer bevorzugten Ausführungsform der vorliegenden Erfindung ist die wenigstens eine externe Funktionseinrichtung kraft- und/oder formschlüssig an die Anordnung ankoppelbar zum funktionellen Ankoppeln der externen Funktionseinrichtung an einen Koppelpartner auf der Seite der Anordnung.

Das "funktionelle Ankoppeln" der externen Funktionseinrichtung an einem Koppelpartner auf der Seite der Anordnung meint, dass die wenigstens eine externe Funktionseinrichtung und der wenigstens eine Koppelpartner auf der Seite der Anordnung zum Ausüben einer Funktion, wie beispielsweise zur Übertragung von Messwerten, miteinander verbunden werden.

Ein solches Ankoppeln kann durch direktes Verbinden der wenigstens einen externen Funktionseinrichtung mit dem Koppelpartner und/oder durch Verbinden mittels einer elektrischen Leitung, einem Kabel zur Datenübertragung und/oder kabellosen Verbindung, wie beispielsweise Infrarot, Bluetooth, WLAN, RFID (Radio Frequency Identification, Identifizierung mit Hilfe von elektromagnetischen Wellen) und dergleichen erfolgen. Es können weitere Einrichtungen zum Ankoppeln bzw. funktionellen Verbinden vorgesehen sein.

Das Ankoppeln kann ferner formschlüssig oder kraftschlüssig berührend, magnetisch oder elektromagnetisch anziehend bzw. abstoßend, unterdruck- oder vakuumangesaugt erfolgen, usw. Entsprechende Einrichtungen können vorgesehen sein.

In einer weiter bevorzugten Ausführungsform der vorliegenden Erfindung kann die erfindungsgemäße Verbindungsvorrichtung wenigstens ein fest an der Anordnung integrierbares Trägerelement und/oder Stützelement aufweisen.

Ein "Trägerelement" kann ein Koppelpartner und/oder ein Außenträger und/oder ein Innenträger der Anordnung sein.

Ein "Stützelement" kann ein Verbindungsträger, ein unterer Dübelanschlag, wie er exemplarisch in der beigefügten Zeichnung und ihrer Beschreibung erläutert ist, ein Öffnungsanschlag, ein oberer Dübelanschlag und/oder ein Kopplungsanschlag sein.

Ein solches Stützelement kann zum Begrenzen einer Bewegung der beweglichen Einrichtungen der erfindungsgemäßen Vorrichtung geeignet sein.

Fest integrierte Einrichtungen verschleißen vorteilhafter Weise weniger. Sie können als Gehäuse, zum Schutz der erfindungsgemäßen Verbindungsvorrichtung, zum Aufnehmen des Ankoppelpartners und dergleichen fungieren. Die Stützelemente können ferner ein Herausrutschen der beweglichen Einrichtungen verhindern.

In einer bevorzugten Ausführungsform der vorliegenden Erfindung ist wenigstens ein weiterer Kontaktabschnitt, beispielsweise ein Außenträger und/oder ein Koppelpartner, fest an der Anordnung integrierbar.

In einer weiter bevorzugten Ausführungsform der vorliegenden Erfindung ist die zweite Stellung eine Arbeitsstellung.

In der "Arbeitsstellung" im Sinne der vorliegenden Erfindung ist die wenigstens eine externe Funktionseinrichtung mit der Anordnung zu ihrem bestimmungsgemäßen Gebrauch verpresst.

In der Arbeitsstellung kann insbesondere die eigentliche Nutzkopplung zwischen der externen Funktionseinrichtung und dem Koppelpartner stattfinden. Die externe Funktionseinrichtung kann mechanisch spielfrei und unter Kraft an den anordnungsseitigen Koppelpartner angekoppelt sein. Die externe Funktionseinrichtung ist funktionsfähig mit dem Ankoppelpartner verbunden.

In einer weiter bevorzugten Ausführungsform der vorliegenden Erfindung ist die zweite Stellung eine Verschlussstellung. In dieser kann der erste Kontaktabschnitt auf dem weiteren Kontaktabschnitt oder auf einem Abschnitt der Anordnung, z.B. einem Gehäuseabschnitt, aufliegen oder mit diesem eine Abdichtung darstellen.

Der erste Kontaktabschnitt kann in der Verschlussstellung bevorzugt gas- und/oder flüssigkeitsdicht, allgemein besonders bevorzugt fluiddicht, auf dem weiteren Kontaktabschnitt aufliegen.

Der erste Kontaktabschnitt kann ein Deckel sein. Das Aufliegen des ersten Kontaktabschnitts auf dem weiteren Kontaktabschnitt oder das Erzeugen einer Abdichtung mit demselben kann die erfindungsgemäße Vorrichtung vorteilhaft vor dem Eindringen von Schmutz und oder anderen Fremdkörpern in ihr Inneres oder in ein Inneres der Anordnung schützen. Über eine derartige Abdichtung kann vorzugsweise ferner hinweg gewischt oder auf andere Weise gereinigt werden, ohne dass Feuchtigkeit oder dergleichen in das Innere der Anordnung eindringt. Erfindungsgemäß können weitere Einrichtungen zum Abdichten der erfindungsgemäßen Vorrichtung, wie beispielsweise Dichtringe, Schnappverschlüsse und dergleichen, vorgesehen sein.

In einer weiter bevorzugten Ausführungsform der vorliegenden Erfindung ist die externe Funktionseinrichtung ein Einwegprodukt wie ein Schlauch, eine Leitung, ein extrakorporaler Blutkreislauf einer Dialysiervorrichtung oder dergleichen.

In einer weiter bevorzugten Ausführungsform weist die externe Funktionseinrichtung ein Druckmess-Disposable, insbesondere ein Druckmess-Disposable, welches Bestandteil eines Disposable-Schlauchsatzes ist, auf oder ist als ein solches Druckmess-Disposable ausgestaltet. Der Koppelpartner weist einen Drucksensor an einer Blutbehandlungsmaschine zur extrakorporalen Blutbehandlung, insbesondere einer Dialysemaschine, auf oder ist ein solcher Drucksensor. Das Druckmess-Disposable ist zum Messen eines Drucks im Druckmess-Disposable an den Drucksensor ankoppelbar.

Mittels der erfindungsgemäßen Verbindungsvorrichtung ist es in einer weiter bevorzugten Ausführungsform auch möglich, beim Übergang aus der ersten Stellung in die zweite Stellung zunächst mit einer geringeren, exemplarischen, Kraft F₁₁ < F₂₂, und erst im Anschluss hieran mit der größeren, exemplarischen, Kraft F₂₂ > F₁₁ zu arbeiten. Dabei kann ein breiter Einbauspalt, in welchem z.B. ein Finger gequetscht werden könnte, mit einer diesen nicht verletzenden Kraft verringert werden. Analog wird erst bei einer Breite des Einbauspalts, bei welcher z.B. ein Finger nicht mehr in den Einbauspalt gebracht werden kann, mit der größeren Kraft verpresst.

Bevorzugt kann die Geschwindigkeit, mit welcher der Spalt ausgehend von der ersten Stellung, z.B. der Rüststellung, verringert wird, zunächst derart niedrig angesetzt werden, dass genügend Zeit verbleibt, einen versehentlich in den Einbauspalt eingebrachten Finger aus diesem wieder herauszuziehen, bevor die größere Kraft zum Verpressen wirkt.

Die erfindungsgemäße Aufgabe wird ebenso durch eine Anordnung gemäß dem Anspruch 9 gelöst.

Da die erfindungsgemäße Anordnung eine erfindungsgemäße Verbindungsvorrichtung zum Verbinden wenigstens einer externen Funktionseinrichtung mit einer Anordnung aufweist, wird zur Vermeidung von Wiederholungen wiederum auf die vorstehend beschriebenen Ausführungen derselben verwiesen. Mittels der erfindungsgemäßen Verbindungsvorrichtung jeweils erzielbare Vorteile sind ungeschmälert auch durch die erfindungsgemäße Anordnung erzielbar.

Die erfindungsgemäße Anordnung kann jede zum Zwecke der vorliegenden Erfindung geeignete medizintechnische Anordnung, Anordnung aus der Labortechnik oder der Nahrungsmittelherstellung sein. In einer bevorzugten Ausführungsform der vorliegenden Erfindung ist die erfindungsgemäße Anordnung eine Blutbehandlungsvorrichtung.

Die erfindungsgemäße Anordnung kann, wie in einer bevorzugten Ausführungsform vorgesehen, zusätzlich eine Steuer- oder Regeleinrichtung aufweisen, welche geeignet und konfiguriert ist, um eine pneumatisch wirkende oder pneumatisch betreibbare Einrichtung der Verbindungsvorrichtung - z. B. eine Öffnungsaktorbaugruppe - zu steuern oder zu regeln.

Die Steuer- oder Regeleinrichtung kann eine üblicherweise an Blutbehandlungs- oder anderen Anordnungen vorgesehene Einrichtung zum Steuern oder Regeln sein. Sie kann alternativ separat zu dieser vorgesehen sein.

Die Steuer- oder Regeleinrichtung kann eine CPU sein, eine solche aufweisen oder in einer solchen programmiert sein.

Da externe Funktionseinrichtungen in der Praxis regelmäßig mittels Druck gehalten bzw. verpresst werden, besteht die Gefahr, dass die Finger desjenigen, der die externe Funktionseinrichtung einlegt, zwischen den Kontaktabschnitten eingeklemmt werden können. Aus dem Stand der Technik sind verschiedene Sicherheitsvorrichtungen bekannt.

So ist aus der DE 44 19 593 A1 bekannt, eine Kraft zum Verpressen der externen Funktionseinrichtung auf einen Wert zu begrenzen, der für ein Quetschen von Fingern zu niedrig ist. Andere, aus der Praxis bekannte Vorrichtungen weisen einen Sensor auf, welcher das Einführen von Fingern und dergleichen detektiert und meldet.

In der vorliegenden Erfindung erfolgt ein Übergang der Verpresseinrichtung aus einer ersten in eine zweite Stellung, beispielsweise mittels einer ersten und einer zweiten Feder mit verschiedenen Federkräften FE₁ bzw. FE₂, mit welchen eine erste Federkraft F₁ und eine zweite Federkraft F₂ erzielbar sind. Die Federkräfte FE₁ und FE₂ der Federn ergeben sich aufgrund deren Federkennlinien, z.B. ist die lineare Federkennlinie - unter Vernachlässigung von Reibung - definiert als: Federkraft = Federsteifigkeit x Federweg.

In Abhängigkeit vom Abstand der Kontaktabschnitte zueinander ist erfindungsgemäß eine gestufte oder sprungweise Kraftaufbringung erzielbar. Es ist daher möglich, die volle Anpresskraft vorzugsweise und vorteilhaft nur auf einem kurzen Verfahrweg bei weit geöffnetem Einbauspalt aufzubringen, der beispielsweise der Dicke oder dem Durchmesser der externen Funktionseinrichtung entspricht. Das weitere Zufahren der Verbindungsvorrichtung kann vorteilhaft mit verringerter Kraft erfolgen, so dass zum Beispiel keine Finger verletzt werden können, die in den Einbauspalt geraten sind. Hier besteht ein möglicher, mittels der vorliegenden Erfindung erzielbarer Vorteil.

Mit der vorliegenden Erfindung kann es in vorteilhafter Weise ferner möglich sein, eine Anpresskraft der Verbindungsvorrichtung zu erhöhen und so eine verbesserte Ankopplung zu erreichen. Gleichzeitig kann es in vorteilhafter Weise möglich sein, eine Verletzungsgefahr für den Benutzer zu verringern, wie oben beschrieben.

Die vorliegende Erfindung kann somit vorteilhaft die Steigerung der Ankopplungskraft bei keinem oder nur geringem Mehraufwand an Antriebsenergie ermöglichen, da ein Teil des Verpressungsweges unter der geringen Kraft der Verschiebungsfeder zurückgelegt wird. Dies erlaubt eine vergleichsweise vereinfachte Konstruktion der erfindungsgemäßen Verbindungsvorrichtung, gemessen an einer Verbindungsvorrichtung des Standes der Technik, in welcher zwischen einer ersten, geringen Kraft, und einer zweiten, höheren Kraft zum Verfahren der Kontaktabschnitte nicht unterschieden wird.

Da bei den meisten Anwendungen die Ruhezeiten der Anordnung in der Verschlussstellung länger als die Verweilzeiten in Arbeitsstellung sein können, kann die vorliegende Erfindung in vorteilhafter Weise die in der Verschlussstellung nur mehr unter Kraftwirkung der geringeren Kraft der Verschiebungsfeder liegenden Bauteile vor Verformung, Ermüdung, Abnützung und dergleichen schonen.

Die Kraft der Verschiebungsfeder kann so klein gewählt werden, dass sie gerade noch die gewünschten Funktionen der Rückstellung und Abdichtung der Maschinenanordnung übernimmt. Somit sind die potentiellen Quetschkräfte, Verschiebungskräfte und Ruhebelastungskräfte in vielen Fällen vorteilhaft kleiner wählbar als bei einer herkömmlichen Anordnung mit nur einer Kraftstufe, welche im Hinblick auf die gerade noch zulässige oder erforderliche Verpresskraft gewählt wurde. Letzteres kann in vorteilhafter Weise zu einem geringeren Bauraumbedarf führen.

Ferner kann in der Verschlussstellung der vorliegenden Erfindung, in der keine externe Funktionseinrichtung verpresst bzw. eingebaut ist, mittels der erfindungsgemäßen Verbindungsvorrichtung der Einbauraum und gegebenenfalls auch weitere Räume vorteilhaft verschlossen werden. Sie kann vorteilhaft ein gutes Reinigungsverhalten der Anordnung sowie einen sicheren Schutz eines Inneren der erfindungsgemäßen Vorrichtung und/oder der Anordnung gegen das Eindringen unerwünschter Partikel und Flüssigkeiten gewährleisten.

Mittels der vorliegenden Erfindung können in vorteilhafter Weise Einbauräume und Kräfte konstruktiv verändert werden. Mit der vorliegenden Erfindung kann es vorteilhaft möglich sein, jeden Funktionsabschnitt entsprechend anzupassen und so optimal wie möglich zu gestalten.

Die vorliegende Erfindung kann durch eine neue Anordnung an sich bekannter mechanischer oder anderer Funktionselemente die oben genannten Anforderungen vorteilhaft auf neue, sichere, einfache und kostensparende Art erfüllen.

Da mittels der vorliegenden Erfindung eine abgestufte Kraftwirkung in Abhängigkeit vom Abstand der Kontaktabschnitte erfolgen kann, kann sich in vorteilhafter Weise die Verwendung anderer Schutzsysteme wie Gitter oder Sensoren oder gesteuerter Sicherheitsaktoren oder komplizierter Eingriffhindernisse erübrigen. Letzteres kann vorteilhaft den technischen Aufwand verringern bei vergleichbarer oder gar erhöhter Sicherheit.

Bei Energie- und Steuerungsausfall kann sich bei der vorliegenden Erfindung - insbesondere da mechanische Federn verwendet werden - in vorteilhafter Weise ein sicherer Ablauf einstellen. Die mögliche Quetschkraft bzw. die Breite des möglichen Quetsch- oder Einbauspalts liegen immer mechanisch fest vorgegeben im sicheren Bereich.

Mit der vorliegenden Erfindung können die Funktionseigenschaften der Anordnung vorteilhaft verbessert werden, da eine Erhöhung der Verpresskraft in der Arbeitsstellung beispielsweise auf das ca. Dreifache des ursprünglichen Wertes erreicht werden kann. Dies kann mittels der abgestuften Kraftwirkung erreicht werden, ohne Änderungen an den grundlegenden Maßverhältnissen der Anordnung und der externen Funktionseinrichtung und/oder an der energetischen Auslegung der Anordnung vornehmen oder eine Gefährdung des Personals in Kauf nehmen zu müssen.

Die potentielle Quetschkraft von in den Einbauspalt einer Rüststellung eingeführten Körperteilen, insbesondere Fingern, kann durch die Verteilung der aufgebrachten Kraft auf verschiedene Kräfte, z.B. eine erste und eine zweite Kraft, in vorteilhafter Weise herabgesetzt werden.

Die vorliegende Erfindung kann in vorteilhafter Weise ein manuelles Einlegen der externen Funktionseinrichtung unter Aufwendung geringen Geschicks und geringer Kraft erlauben.

Die vorliegende Erfindung wird im Folgenden anhand einer bevorzugten erfindungsgemäßen Ausführungsform unter Bezugnahme auf die angehängte, schematisch stark vereinfachte Zeichnung beschrieben. In den Figuren werden gleiche Bezugszeichen zum Bezeichnen gleicher Elemente verwendet. In der Zeichnung gilt:
- Fig. 1: zeigt schematisch den Längsschnitt einer Rüststellung einer ersten Ausführungsform der erfindungsgemäßen Vorrichtung;
- Fig. 2: zeigt schematisch den Längsschnitt einer Arbeitsstellung der Ausführungsform der Fig. 1;
- Fig. 3: zeigt schematisch den Längsschnitt einer Verschlussstellung der Ausführungsform der Fig. 1;
- Fig. 4: zeigt schematisch den Längsschnitt einer Kraftwechselstellung der Ausführungsform der Fig.1;
- Fig. 5: zeigt als Teil-Blockbild eine erfindungsgemäße Anordnung in vereinfachter Darstellung; und
- Fig. 6: zeigt ein Beispiel für ein Kraft-Weg-Diagramm.

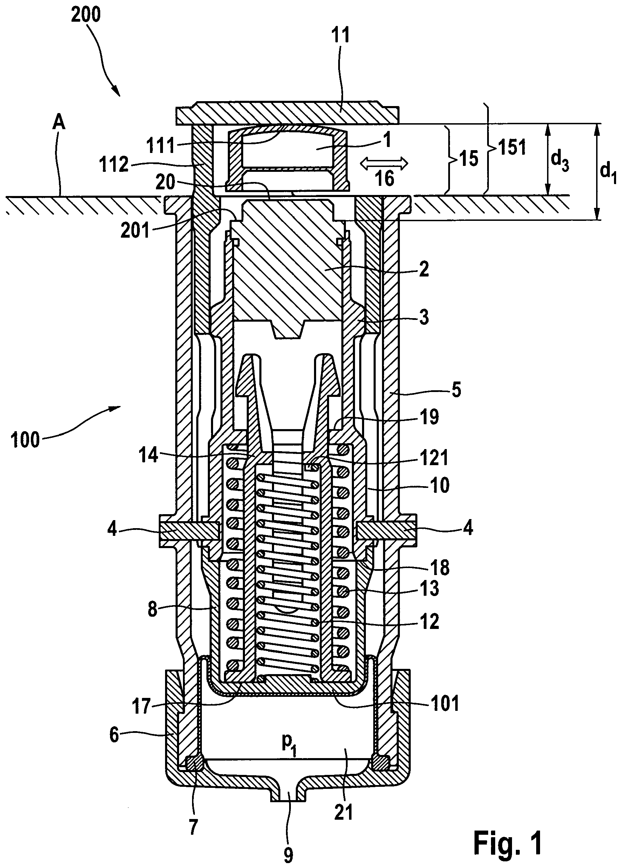

Im Folgenden wird ein Verfahren beschrieben. Es wird dabei auf die vier in den Fig. 1 bis 4 gezeigten Stellungen einer beispielhaften ersten Ausgestaltung bzw. Ausführungsform der erfindungsgemäßen Verbindungsvorrichtung und deren konstruktive Ausgestaltung und die beigefügte Bezugszeichenliste Bezug genommen.

Fig. 1 zeigt eine erfindungsgemäße Verbindungsvorrichtung 100 in der so genannten Rüststellung. Die Rüststellung ist zum "Rüsten" einer Anordnung mit wenigstens einer externen Funktionseinrichtung 1, also zum Einlegen der externen Funktionseinrichtung 1 in eine Aufnahmeeinrichtung 151, insbesondere in eine Öffnung oder einen Spalt bzw. Einbauspalt derselben, geeignet.

Vereinfacht ausgedrückt ist die Verbindungsvorrichtung 100 der Fig. 1 bis 4 aus drei im Wesentlichen zylindrischen, koaxial ineinander angeordneten Hohlkörpern unterschiedlichen Durchmessers aufgebaut. Ein Übergang aus der Rüststellung in eine andere Stellung kann durch eine Verpresseinrichtung bewirkt und durch definierte Anschläge begrenzt werden.

Die Verbindungsvorrichtung 100 weist bewegliche Einrichtungen auf, wie beispielsweise kraft- und/oder bewegungs- und/oder geschwindigkeitsvermittelnde Einrichtungen. Zu ihnen zählen bewegliche Trägerelemente, wie ein Mittelträger 10, eine Öffnungsaktorbaugruppe 6, 7, 8 und 9 (im Folgenden kurz: 6-9) zum Aufbringen einer Kraft und Energiespeicher- und/oder Kraftübertragungseinrichtungen in Form einer Verschiebungsfeder 12 und einer Verpressungsfeder 13.

Die pneumatische Öffnungsaktorbaugruppe 6-9 weist einen Rollbalg 7, einen Druckluftanschluss 9, einen Kolben 8 und einen Zylinder 6 auf.

Die Öffnungsaktorbaugruppe 6-9 kann alternativ auch aus einer bekannten Kolben-Zylinderanordnung (vorzugsweise mit Kolbendichtring) oder aus einem linearen Getriebe (etwa mit elektrischem Antrieb) bestehen oder solche aufweisen.

Die Öffnungsaktorbaugruppe 6-9 kann ausgestaltet sein, um nur in die Öffnungsrichtung der Verbindungsvorrichtung 100 antreibend zu wirken.

Die Verschiebungsfeder 12 und die Verpressungsfeder 13 lassen sich funktionell zu einer Verpresseinrichtung zählen, mittels welcher die externe Funktionseinrichtung 1 verpressbar ist. Die Verpresseinrichtung kann weitere Elemente aufweisen.

Die Öffnungsaktorbaugruppe 6-9 kann ausgestaltet sein, um in der Schließrichtung der Verpresseinrichtung nur passiv und/oder gebremst beweglich zu sein.

Zum Verpressen wird wenigstens eine erste Federkraft F₁ und eine zweite Federkraft F₂ mittels der Federn 12, 13 und optional der Öffnungsaktorbaugruppe 6-9 auf das bewegliche Trägerelement 10 mit einem Boden 101 übertragen oder aufgebracht.

Fig. 1 zeigt drei ineinander angeordnete Hohlkörper. Der äußerste der drei Hohlkörper wird als Außenträger 5 bezeichnet. Er kann stirnseitig starr, d.h. fest integriert, im Inneren einer Anordnung 200, beispielsweise einer Blutbehandlungsmaschine, auf Höhe einer Ankoppelfläche A zum Ankoppeln einer externen Funktionseinrichtung 1 angeflanscht sein.

Die Ankoppelfläche A ist ein Abschnitt auf der Oberseite eines Außenträgers 5. Sie kann allerdings auch eine äußere Sensoroberfläche oder ein Vorsprung 201 eines mit dem Innenträger 3 fest integrierten Sensors 2 oder jeder andere zu einer Kraftaufnahme geeignete Abschnitt sein.

Im Inneren des Außenträgers 5 wird mittels starrer radialer Verbindungsträger 4 der im Wesentlichen zylindrische Innenträger 3 koaxial zum Außenträger 5 gehalten. Der vom Innenträger 3 getragene Sensor 2 ist gegenüber der Ankoppelfläche A stets starr in derselben Position angeordnet.

Zwischen dem Außenträger 5 und dem Innenträger 3 ist ein freier, gleichmäßiger ring- oder rohrförmiger Spalt vorgesehen. Das in den Figuren jeweils unten dargestellte freie Ende des Außenträgers 5 ist durch einen Boden geschlossen und weist einen Druckanschluss 9 auf.

Als weitere, ineinander liegende Hohlkörper sind ein Mittelträger 10 und ein Federdübel 14 vorgesehen. Diese beiden Hohlkörper sind jeweils und getrennt voneinander axial innerhalb des Außenträgers 5 verschiebbar.

Der Mittelträger 10 befindet sich in der maximal ausgefahrenen Stellung, limitiert durch den Öffnungsanschlag 18, der ein weiteres Ausfahren verhindert. Die Verpressungsfeder 13 ist gespannt und hält den Federdübel 14 am unteren Dübelanschlag 17 fest an den Boden 101 des Mittelträgers 10 gedrückt. Da die Verpressungsfeder 13 stärker als die Verschiebungsfeder 12 ist, wird die Verschiebungsfeder 12 auf eine definierte Länge, nämlich auf die Länge des Innenraums im Federdübel 14, zusammengedrückt. Die Verschiebungsfeder 12 wirkt dabei der Verpressungsfeder 13 entgegen. Insgesamt wirkt resultierend eine Kraft F_{2, Rüst} als Verpresskraft.

Der Mittelträger 10 ist an einer seiner Stirnseiten fest mit einer Andruckplatte 11 verbunden. Die Andruckplatte 11 ist mit variablem Abstand von der Ankoppelfläche A beabstandet. In Fig. 1 beträgt der Abstand d₃. Zwei Kontaktabschnitte 111 und 201 stehen in einem ersten Abstand d₁ zueinander. Bei der in den Figuren gezeigten Ausführungsform könnte auch die Ankoppelfläche A als Kontaktabschnitt wirken. Dennoch ist im Folgenden vornehmlich von der entsprechenden Fläche der Andruckplatte 11 als weiterer Kontaktabschnitt 111 die Rede.

Der bewegliche Mittelträger 10 weist an seinem Umfang Durchbrüche auf, durch welche Verbindungsträger 4 in das Innere des Mittelträgers 10 hineinragen. Der Mittelträger 10 ist im ringförmigen Spalt zwischen dem Außenträger 5 und dem Innenträger 3 begrenzt axial verschiebbar.

Im Inneren des Mittelträgers 10 ist der Federdübel 14 angeordnet. Der Federdübel 14 kann axial zu einem Inneren des Innenträgers 3 verschoben werden. Der Mittelträger 10 ist an seiner anderen Stirnseite (in Fig. 1 als unteres Stirnende dargestellt) durch seinen Boden 101 verschlossen.

Die Andruckplatte 11, ein Abschnitt 112 des Mittelträgers 10 und die Oberseite des Sensors 2 bzw. ein Abschnitt 201 des Sensors 2 sind Teil der hier C-förmig ausgestalteten Aufnahmeeinrichtung 151.

Im Bereich des Bodens des Außenträgers 5 ist die Öffnungsaktorbaugruppe 6-9 angeordnet.

Die Öffnungsaktorbaugruppe 6-9 weist einen luftdichten Rollbalg 7 auf. Dieser kann derart umlaufend abdichtend angeordnet sein, dass zwischen dem Boden 101 und dem Rollbalg 7 ein pneumatischer Arbeitsraum 21 entsteht. Der Rollbalg 7 liegt außen auf dem Boden 101 des Mittelträgers 10 von unten auf. Er dichtet den Arbeitsraum 21 gegen den Ringspalt zwischen dem Mittelträger 10 und dem Außenträger 5 ab.

Zum Aufnehmen einer externen Funktionseinrichtung 1 wird über den Druckluftanschluss 9 Luft, ein anderes Gas oder ein anderes Fluid mit einem ersten Druck p₁ in den Arbeitsraum 21 gepresst. Der Rollbalg 7 drückt gegen den Boden 101 des Mittelträgers 10 und verschiebt den Mittelträger 10 - in Abhängigkeit vom ersten Druck p₁ - axial aus dem Außenträger 5 heraus.

Die Andruckplatte 11 hebt und entfernt sich von der Ankoppelfläche A. Die Andruckplatte 11 und die Ankoppelfläche A sind daraufhin mit einem oben erwähnten Abstand d₃ beabstandet, wie dies in Fig. 1 zu erkennen ist. Der Abstand d₃ bildet eine Öffnung oder einen Spalt bzw. Einbauspalt 15 zum Aufnehmen einer externen Funktionseinrichtung 1.

Die externe Funktionseinrichtung 1 kann durch eine mit Pfeil markierte, in Fig. 1 horizontal ausgeführte Bewegung 16 in den Einbauspalt 15 eingesetzt werden. Zwischen den Kontaktabschnitten 111 und 201 für die externe Funktionseinrichtung ergibt sich in dieser Stellung der oben erwähnte Abstand d₁.

Der Federdübel 14 kann durch die beiden Federn 12 und 13 gegen den Mittelträger 10 und den Innenträger 3 gelagert oder abgestützt sein. Die stärkere und größere Feder 13 wird aufgrund ihrer Funktion als Verpressungsfeder 13 mit einer Federkraft FE₁, und die schwächere und kleinere Feder 12 aufgrund ihrer Funktion als Verschiebungsfeder 12 mit einer Federkraft FE₂ bezeichnet.

Aufgrund der verwickelten Kraftverhältnisse aufgrund beispielsweise elastischer Verformungen der betroffenen Bauteile und Anschläge Richtung der Kraftwirkung der Federn und dergleichen, handelt es sich i.d.R. in den Ausführungsbeispielen nicht um die freigeschnittenen Federkräfte FE₁ und FE₂ der Verschiebungsfeder 12 und der Verpressungsfeder 13.

Die Federn 12 und 13 können allgemein in jeder Stellung der Verbindungsvorrichtung mit einer Vorspannung beaufschlagt sein. Das Ausmaß ihrer jeweiligen Vorspannung kann dabei in Abhängigkeit von der jeweiligen Stellung der Verbindungsvorrichtung variieren.

Die Verschiebungsfeder 12 ist im Inneren des Federdübels 14 mit einem ihrer Enden gegen einen Anschlag 121 gelagert und drückt mit ihrem anderen Ende gegen die Innenseite des Bodens 101 des Mittelträgers 10. Das dem Boden 101 des Mittelträgers 10 zugewandte Ende des Federdübels 14 weist einen Außenflansch auf.

Das andere Ende des Federdübels 14 ist als federnde Schnappvorrichtung ausgeführt. Sie kann bei der Montage der erfindungsgemäßen Verbindungsvorrichtung 100 an der Anordnung 200 in der Öffnung eines oberen Dübelanschlags 19 des Innenträgers 3 verrastet werden.

Durch die Montage der Verschiebungsfeder 12 wird einerseits aufgrund der Toleranzen zwischen dem Außendurchmesser der Verschiebungsfeder 12 und dem Innendurchmesser des Federdübels 14 im Bereich der Verschiebungsfeder 12 ("Restspalt") die Schnappvorrichtung, mit welcher der Federdübel 14 im Innenträger 3 verrastet wird, gegen Zusammendrücken gesichert. Dadurch wird gewährleistet, dass es nicht zu einem unbeabsichtigten Lösen der Schnappverbindung durch Zusammendrücken des Federdübels 14 oder einer andauernden Biegeverformung des Federdübels 14 im Betrieb kommt.

Andererseits ist der Restspalt derart montage- und demontagegerecht ausgeführt, dass Reibung zwischen dem Außendurchmesser der Verschiebungsfeder 12 und dem Innendurchmesser des Federdübels 14 über die Länge der Verschiebungsfeder 12 verhindert wird.

Grundsätzlich reicht es aus, wenn die Verschiebungsfeder 12 nur mit ihrem ringförmigen Ende den Federdübel 14 durch Formschluss gegen radiales Zusammendrücken sichert. Dazu kann die aufnehmende Auflagefläche des oberen Endes der Verschiebungsfeder 12 im Federdübel 14 als kurzer gestufter Absatz ausgeführt sein, in den das Ende der Feder derart eingepasst ist, dass der Restspalt ansonsten relativ breit ausgeführt werden kann.

Es ist allerdings auch möglich, den Innenteil des Federdübels 14 im Wesentlichen über dessen gesamte oder im Wesentlichen gesamte Länge oder über einen Abschnitt hiervon konisch auszuführen derart, dass die Verschiebungsfeder 12 zwar leicht montiert und demontiert werden kann, jedoch trotzdem ein unbeabsichtigtes Lösen der Schnappverbindung verhindert wird. Das obere Ende der Verschiebungsfeder 12 ist dann im konisch eng zulaufenden Teil des Konus im Federdübel 14 gelagert und verhindert ein Zusammendrücken des Federdübels 14.

Die Verpressungsfeder 13 umgibt Abschnitte des Federdübels 14. Sie drückt mit einem Ende gegen den Außenflansch des Federdübels 14 und mit dem anderen Ende gegen den oberen Dübelanschlag 19 des Innenträgers 3.

Die Verschiebungsfeder 12 und die Verpressungsfeder 13 können mit Hilfe der Öffnungsaktorbaugruppe 6-9 gestaucht werden.

Die in Fig. 1 gezeigte Rüststellung wird mittels der Öffnungsaktorbaugruppe 6-9 erzielt. Während des Übergangs in die in Fig. 1 gezeigte Rüststellung überwindet die Öffnungsaktorbaugruppe 6-9 die ihr entgegenwirkenden Kräfte der Federn 12, 13 und die Reibungskräfte zwischen ruhenden Bauteilen 2, 3, 4 und 5 und beweglichen Bauteilen 7, 8, 10, 11, 12, 13 und 14 der erfindungsgemäßen Verbindungsvorrichtung 100. In der Rüststellung sind die beiden Federn 12, 13 maximal zusammengedrückt.

Wenn der Mittelträger 10 an einem Öffnungsanschlag 18 des Innenträgers 3 anstößt, wird die Öffnungsbewegung des Einbauspalts 15 beendet. Der Federdübel 14 ist vom oberen Dübelanschlag 19 des Innenträgers 3 maximal nach oben abgehoben.

Während des manuellen Einsetzens (oder auch Herausnehmens) der externen Funktionseinrichtung 1 aus dem Einbauspalt 15 bleibt die Öffnungsaktorbaugruppe 6-9 aktiv eingeschaltet.

Die Öffnung bzw. der Einbauspalt 15 der Aufnahmeeinrichtung 151 kann jedoch auch - anders als in den Figuren gezeigt - ohne Wirkung oder Vorhandensein der Öffnungsaktorbaugruppe 6-9 erfolgen. Hierzu kann sich der Benutzer der Anordnung 200 beispielsweise seiner Finger bedienen. Er kann die Aufnahmeeinrichtung 151 manuell aufziehen. Zur besseren Handhabung bzw. zum besseren Eingriff mit dem oder den Fingern kann eine Halteeinrichtung wie ein Griff, eine Lasche, ein Ring oder dergleichen vorgesehen sein. Diese Halteeinrichtung (nicht gezeigt) kann an der äußeren Oberfläche der Andruckplatte 11 befestigt sein. Diese Ausgestaltung stellt eine besonders kostengünstige Ausführungsform der vorliegenden Erfindung dar. Ferner erlaubt sie vorteilhaft das Bedienen, Auf- oder Abrüsten der Anordnung 200 auch im Falle eines Stromausfalls. Sie kann somit auch ergänzend zum Ermöglichen einer Notfunktion vorgesehen sein.

In der Rüststellung ist der Einbauspalt 15 groß genug, ein ergonomisch günstiges Einsetzen und Herausnehmen der externen Funktionseinrichtung 1 zu erlauben. Aufgrund der Größe des Einbauspalts 15 können allerdings auch beispielsweise Finger einer Bedienperson ungewollt in diesen geraten.

In Fig. 1 sind ferner ein unterer Dübelanschlag 17 und ein Kopplungsanschlag 20 gezeigt.

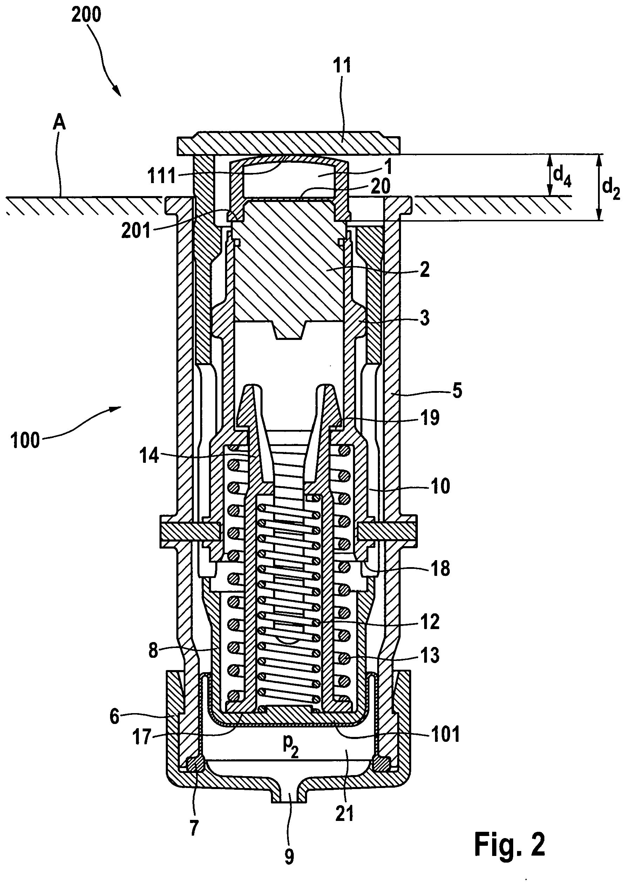

Um die externe Funktionseinrichtung 1 mit der Anordnung zu verpressen und an diese anzukoppeln, wird die erfindungsgemäße Vorrichtung in die Arbeitsstellung verfahren, wie sie in Fig. 2 gezeigt ist.

Die Andruckplatte 11 und die Ankoppelfläche A sind in der Arbeitsstellung mit einem Abstand d₄ beabstandet, wie dies in Fig. 2 zu erkennen ist. Zwischen dem ersten Kontaktabschnitt 111 und dem weiteren Kontaktabschnitt 201 zur Aufnahme der externen Funktionseinrichtung 1 ergibt sich in dieser Stellung der zweite Abstand d₂.

Die Arbeitsstellung der erfindungsgemäßen Verbindungsvorrichtung 100 wird nach Passivschalten der Öffnungsaktorbaugruppe 6-9 angefahren bzw. erreicht. Die Arbeitsstellung kann durch Lösen des mit dem Finger - in der zu Fig. 1 alternativ diskutierten Ausführungsform - aufgebrachten Zugs erreicht werden.

Die beiden Federn 12 und 13 treiben die beweglichen Bauteile 7, 8 der erfindungsgemäßen Verbindungsvorrichtung 100 gegen vorhandene Reibungswiderstände an.

Im Arbeitsraum 21 wird ein pneumatischer zweiter Druck p₂ angelegt. Der zweite Druck p₂ ist kleiner als der in der Rüststellung der Fig. 1 herrschende erste Druck p₁. Der Mittelträger 10 wird in den Arbeitsraums 21 hineingedrückt, bis die Summe der Federkräfte FE₁ und FE₂ von der externen Funktionseinrichtung 1 aufgenommen ist und die Arbeitsstellung am Kopplungsanschlag 20 an einem Abschnitt der Oberseite des Außenträgers 5 bzw. einer Oberfläche des Sensors 2 erreicht ist.

Gegenüber der Rüststellung aus Fig. 1 befindet sich der Mittelträger 10 in einer weniger weit ausgefahrenen Stellung. Die Verpressungsfeder 13 ist weniger stark zusammengedrückt als in der Rüststellung (Fig. 1). Der Mittelträger 10 ist vom Öffnungsanschlag 18 des Innenträgers 3 abgehoben. Wie in der Rüststellung wird der Federdübel 14 von der Verpressungsfeder 13 am unteren Dübelanschlag 17 fest an den Boden 101 des Mittelträgers 10 gedrückt. Die Verschiebungsfeder 12 ist wie in der Rüststellung (Fig. 1) auf die Länge des Innenraums im Federdübel 14 zusammengedrückt. Der Federdübel 14 liegt nicht am oberen Dübelanschlag 19 an. Die Verschiebungsfeder 12 wirkt der Verpressungsfeder 13 entgegen. Insgesamt wirkt resultierend die Kraft F₂ als Verpresskraft. Die Kraft F_{2, Arbeit} ist in der Arbeitsstellung aufgrund der flachen Kennlinie (Kraft-Weg-Diagramm, siehe Fig. 6) nur geringfügig kleiner als bei der Rüststellung (Fig. 1).

Der Federdübel 14 verbleibt am unteren Dübelanschlag 17, bis die Arbeitsstellung und die Ankopplung am Kopplungsanschlag 20 erreicht sind.

Die Kraftübertragung erfolgt derart, dass die Ankoppelkraft zwischen der externen Funktionseinrichtung 1 und einem Kopplungspartner 2, wie beispielsweise einem Sensor, auf der Anordnungsseite oder dem zweiten Kontaktabschnitt 201 von Null auf einen Wert etwas unterhalb der Vorspannkraft der Verpressungsfeder 13 ansteigt, während die Kraft auf die Öffnungsaktorbaugruppe 6-9 bis auf den reibungs- und restvorspannungsbedingten Differenzwert absinkt.

Um ein sicheres Verpressen auch unter Berücksichtigung möglicher Toleranzen und Nachgiebigkeiten der Komponenten der externen Funktionseinrichtung 1 und der Komponenten der erfindungsgemäßen Vorrichtung oder der Anordnung zu gewährleisten, verbleibt ein vorgesehener Reserveweg in Verpressungsrichtung, der am Abstand der beiden Anschlagpartner des oberen Dübelanschlags 19 in Fig. 2 zu erkennen ist.

Nach erfolgter Nutzung der externen Funktionseinrichtung in der Arbeitsstellung, beispielsweise nach Abschluss einer medizinischen Behandlung, fährt die Öffnungsaktorbaugruppe 6-9 die beweglichen Bauteile 11, 12, 13 und 14 der erfindungsgemäßen Verbindungsvorrichtung 100 unter der entgegenwirkenden Kraft wenigstens der Verpressungsfeder 13 und der Reibungskräfte wieder in die Rüststellung. Die externe Funktionseinrichtung 1 kann aus dem Einbauspalt 15 entnommen werden.

Die schwächer ausgelegte und vorgespannte Verschiebungsfeder 12 bleibt während des Rüst-Arbeits-Zyklus der erfindungsgemäßen Verbindungsvorrichtung 100 permanent auf ihrem annähernd konstanten Vorspannungswert.

Der untere Dübelanschlag 17 gerät nicht außer Eingriff bzw. wird nicht vom Boden 101 gelöst oder beabstandet. Die Verschiebungsfeder 12 verbleibt in einer konstanten Lage form- und kraftschlüssig in ihrem Einbauraum eingeschlossen.

Das Bedienpersonal kann sich bei Vorrichtungen des Standes der Technik vor allem beim Übergang von der Rüststellung in eine Verschlussstellung oder beim Übergang von der Rüststellung in die Arbeitsstellung bei nicht in den Einbauspalt 15 eingelegter externer Funktionseinrichtung 1 verletzen.

Der Weg von der Rüststellung in eine Verschlussstellung wird im Rahmen der vorliegenden Beschreibung unterteilt in zwei Bewegungsphasen. Die erste kennzeichnet den Übergang von der Rüststellung in eine Kraftwechselstellung. Die zweite kennzeichnet den Übergang von der Kraftwechselstellung in die Verschlussstellung.

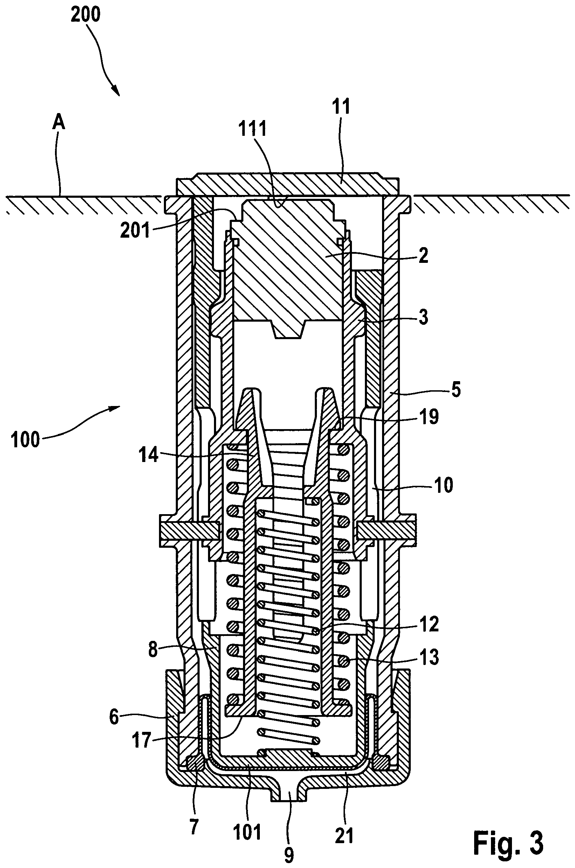

Fig. 3 zeigt eine erfindungsgemäße Verbindungsvorrichtung 100 in der so genannten Verschlussstellung.

Die beiden Federn 12, 13 weisen jeweils eine maximal mögliche Auslenkung auf. Im Arbeitsraum 21 ist der pneumatische Überdruck möglichst gering und geht vorzugsweise gegen Null. Damit kann ein großer Anteil der verbleibenden Vorspannung der Verschiebungsfeder 12 als Dichtkraft zwischen der Andruckplatte 11 und dem Außenträger 5 genutzt werden. Dies kann vorteilhaft zum Erzielen einer möglichst niedrigen Auslegungskraft verwendet werden, was wiederum eine vereinfachte, weniger massive und weniger teure Konstruktion erlaubt.

Während des Übergangs in die Verschlussstellung wird der Mittelträger 10 von der Verschiebungsfeder 12 in den Arbeitsraum 21 hinein gedrückt. Der Arbeitsraum 21 weist ein minimales Volumen auf.

In der Verschlussstellung befindet sich der Mittelträger 10 in vollständig eingefahrener Position. Der untere Dübelanschlag 17 ist vom Boden 101 des Mittelträgers 10 abgehoben. Die Verpressungsfeder 13 dehnt sich so weit aus, dass der obere Dübelanschlag 19 am feststehenden Innenträger 3 angedrückt wird. Sobald der obere Dübelanschlag 19 am feststehenden Innenträger 3 angedrückt wird und der Mittelträger 10 weiter eingefahren wird, dehnt sich die Verschiebungsfeder 12 aus dem Innenraum des Federdübels 14 heraus aus. In der Verschlussstellung wird die Kraft der Verpressungsfeder 13 vollständig vom Federdübel 14 aufgenommen und führt zu entsprechenden elastischen Verformungen des Federdübels 14. Die Verpressungsfeder 13 wirkt in dieser Stellung nicht direkt auf den Mittelträger 10. Nur die Verschiebungsfeder 12 wirkt direkt auf den Boden 101 des Mittelträgers 10. Resultierend wirkt die kleinere Kraft F_{1, Verschluss} zwischen Andruckplatte 11 und Ankoppelfläche A.

Zwischen der Innenseite des Bodens 101 des Mittelträgers 10 und dem Außenflansch des Federdübels 14 befindet sich ein garantierter Freiraum, damit beim Öffnungshub das Arbeitsfluid seinen Druck auf die gesamte Kolbenfläche ausüben kann. Der Federdübel 14 liegt am oberen Dübelanschlag 19 des Innenträgers 3 auf.

Die Andruckplatte 11 berührt im Wesentlichen die Ankoppelfläche A, hier einen Abschnitt der Oberseite des Außenträgers 5.

Die vorliegende Stellung der Fig. 3 wird als Verschlussstellung bezeichnet, da die Andruckplatte 11 den Zugang zum Sensor 2 oder einem Inneren der erfindungsgemäßen Verbindungsvorrichtung 100 oder der Anordnung 200 gegenüber der Umgebung verschließt. Hierbei kann eine Abdichtung der erfindungsgemäßen Verbindungsvorrichtung 100 gegenüber einem Äußeren der Anordnung 200 erzielt werden.

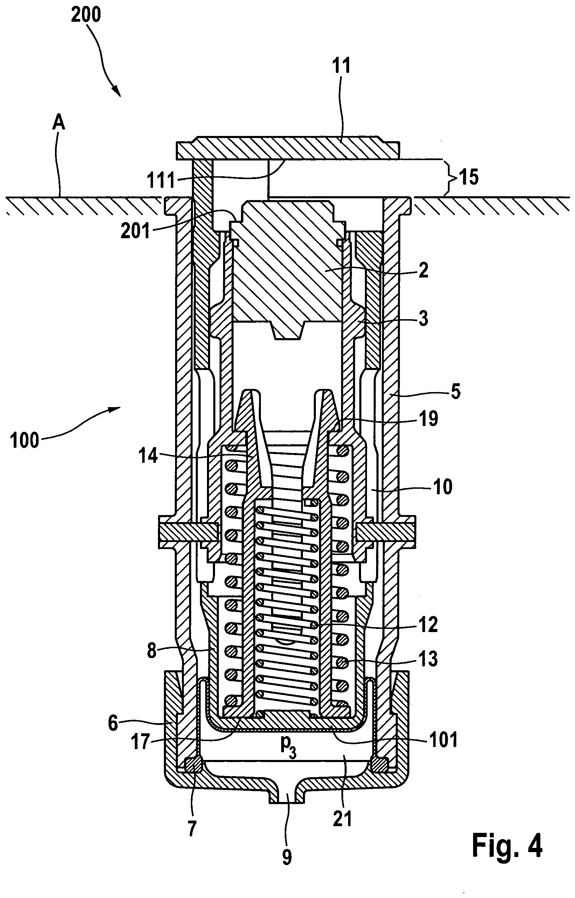

Während des Verfahrens bzw. des Übergangs in die Verschlussstellung durchläuft die erfindungsgemäße Verbindungsvorrichtung 100 eine Kraftwechselstellung, wie sie in Fig. 4 gezeigt ist. In der Kraftwechselstellung der Fig. 4 ist der dritte Druck p₃ im pneumatischen Arbeitsraum 21 größer als Null. Für den Druck werden i.d.R. Drücke im Überdruckbereich in Bezug auf den Umgebungsdruck angewandt.

Die pneumatische Kraft, die auf den Boden 101 des Mittelträgers 10 wirkt, überschreitet gerade die Federkraft FE₂ der Verschiebungsfeder 12. Die Verschiebungsfeder 12 ist maximal zusammengedrückt.

Der Außenflansch des Federdübels 14 liegt am unteren Dübelanschlag 17 auf dem Boden des Mittelträgers 10 auf. Der dritte Druck p₃ ist gerade so groß, dass die Verpressungsfeder 13 eine durch das Anliegen des Federübels 14 am oberen Dübelanschlag 19 vorgegebene Länge und Vorspannung beibehält. Für eine weitere Verschiebung des Mittelträgers 10 aus der Kraftwechselstellung in Richtung zur Verschlussstellung ist der pneumatische Druck p₃ gerade so weit zu senken, dass die vorgespannte Verschiebungsfeder 12 sich weiter ausdehnen kann.

Es herrscht zunächst eine Kraft F₂ die bei weiterem Verfahren der Verbindungsvorrichtung in die Arbeitsstellung sprungartig abfällt und auf eine sehr viel kleinere Kraft F₁ abnimmt. Dieser Verlauf ist beispielsweise im beigefügten Kraft-Weg-Diagramm der Fig. 6, in einer Betrachtung von rechts nach links, veranschaulicht.

Bei der Kraftwechselstellung handelt es sich um eine Übergangsstellung, die beim Wechsel von der Rüststellung zur Verschlussstellung und umgekehrt durchlaufen wird. Wenn der Wechsel von der Verschlussstellung zur Rüststellung hin erfolgt, d.h. der Mittelträger 10 beginnend bei der Verschlussstellung (Fig. 3) ausgefahren wird, so wird die Verschiebungsfeder 12 zunehmend zusammengedrückt und ein Betriebspunkt erreicht, bei dem weiterhin der obere Dübelanschlag 19 am Innenträger 3 anliegt und der untere Dübelanschlag 17 gerade eben Kontakt mit dem Boden 101 des Mittelträgers 10 erhält. Dieser Betriebspunkt ist im Kraft-Weg-Diagramm durch einen ersten Knick 41 dargestellt. Die Kraft der Verpressungsfeder 13 wird in diesem Betriebspunkt noch vollständig zwischen Federdübel 14 und Innenträger 3 aufgenommen und wirkt noch nicht direkt auf den Mittelträger 10. Nur die Kraft der Verschiebungsfeder 12 wirkt direkt auf den Mittelträger 10. Resultierend wirkt eine kleine Kraft F_{1, Kraftwechsel} auf ein mögliches Objekt (z.B. Finger) zwischen Andruckplatte 11 und Ankoppelfläche A. Diese Kraft ist nicht in der Lage, möglicherweise zwischen Andruckplatte 11 und Ankoppelfläche A befindliche Köperteile wie Finger zu verletzen.

Beim weiteren Ausfahren des Mittelträgers 10 über den beschriebenen Betriebspunkt hinaus bewirkt das elastische Bauteilverhalten aller Bauteile, z.B. des Federdübels 14, des Mittelträgers 10 und des Innenträgers 3, des Außenträgers 5 zusammen mit den Federn 12 und 13 in Summe einen kurzen Bereich des steilen Anstiegs im Kraft-Weg-Diagramm bei zunehmendem Ausfahren des Mittelträgers 10. In diesem Bereich wird zunehmend Kraft auf den Boden 101 des Mittelträgers 10 übertragen und der Federdübel 14, der am oberen Dübelanschlag 19 vom Innenträger 3 abhebt, zunehmend entlastet. Der Übergang in den Kräftebereich F₂ ist im Kraft-Weg-Diagramm als zweiter Knick 43 dargestellt.

Wird während der ersten Bewegungsphase des Übergangs von der Rüststellung in die Verschlussstellung oder des Übergangs von der Rüststellung in die Arbeitsstellung ein hinreichend großes Objekt in den Einbauspalt 15 eingeführt, kommt die Schließbewegung an diesem Objekt zum Stillstand. Der Einbauspalt 15 ist in dieser ersten Bewegungsphase jedoch so groß gewählt, dass versehentlich eingeführte Finger noch nicht schmerzhaft gequetscht werden können.

Die Kraftwirkung auf das eingeführte Objekt bzw. Finger durch die Vorspannkraft der Verpressungsfeder 13, gegebenenfalls vermindert um die Reibungswiderstände der erfindungsgemäßen Vorrichtung 100, kann einen Finger aufgrund der verbleibenden Spaltbreite nicht ernsthaft verletzen.

Die Kraftwechselstellung nach Fig. 4 liegt bei Betrachtung eines Übergangs von der Rüststellung in Richtung zur Verschlussstellung um den oben genannten Reserveweg nach der Arbeitsstellung und weist einen entsprechend verminderten Einbauspalt 15 auf. In der Kraftwechselstellung kommt der obere Dübelanschlag 19 in Eingriff bzw. der Federdübel 14 liegt auf diesem auf. Dies ist mit einem Anstieg der Kraft auf diesen oberen Dübelanschlag 19 von Null auf den Vorspannwert der Verpressungsfeder 13 verbunden. Die Verpressungsfeder 13 wirkt jetzt nur mehr innerhalb eines mechanisch geschlossenen Raums aus Federdübel 14 und Innenträger 3.

Während der zweiten Bewegungsphase - von der Kraftwechselstellung in die Verschlussstellung - kann nun die schwächer vorgespannte Verschiebungsfeder 12 wirksam werden. Sie sorgt durch ihre Kraft FE₂ für die Überwindung der Reibungswiderstände der erfindungsgemäßen Verbindungsvorrichtung 100 und insbesondere der Öffnungsaktorbaugruppe 6-9 für die weitere Schließbewegung bis in die Verschlussstellung, wie sie Fig. 3 dargestellt ist.

Die Vorspannkraft der Verschiebungsfeder 12 ist so gewählt, dass sie in Verbindung mit der Formgebung und/oder der Größe des während dieser Bewegungsphase vorhandenen Einbauspalts 15 keine schmerzhaften Verletzungen an Fingern, wie Quetschungen, hervorrufen kann.

Andererseits ist die gewählte Vorspannkraft der Verschiebungsfeder 12 groß genug, um mittels der in der Verschlussstellung wirksamen Abdichtkraft zwischen dem Dichtrand der Andruckplatte 11 und dem Dichtrand des Außenträgers 5 in Verbindung mit geeigneten Dichtelastomeren für eine ausreichende Dichtheit der geschlossenen Anordnung gegen das Eindringen von Partikeln und Flüssigkeiten sorgen zu können.

Im Folgenden wird ein zweites Verfahren unter Bezugnahme auf die vier maßgeblichen Stellungen der erfindungsgemäßen Verbindungsvorrichtung wie in den Fig. 1 bis 4 beschrieben. Das zweite Verfahren entspricht in seiner Ausgestaltung dem vorstehend beschriebenen Verfahren. Zum zweiten Verfahren werden im Folgenden beispielhafte Abmessungen zur Ausgestaltung der erfindungsgemäßen Verbindungsvorrichtung 100 oder von Abschnitten hiervon angegeben.

In einer Rüststellung der erfindungsgemäßen Verbindungsvorrichtung 100 kann ein Einbauspalt 15 z.B. 18 oder 19 mm (Millimeter) messen. Die Verpressungsfeder 13 weist aufgrund der Anschläge eine minimale Länge von 57 mm auf. Die Verschiebungsfeder 12 kann eine Länge von 55 mm aufweisen. Die Distanz zwischen dem Boden 101 des Mittelträgers 10 und dem Einlass des Druckanschlusses 9 kann 26 mm betragen. Das Volumen des Arbeitsraums 21 nimmt dabei einen maximalen Wert an.

In der Arbeitsstellung der erfindungsgemäßen Verbindungsvorrichtung 100 entspricht die Höhe des Einbauspalts 15 im Wesentlichen der Höhe der - gegebenenfalls nur wenig oder aber stärker verpressten - externen Funktionseinrichtung 1 und beträgt z.B. 11, 12 oder 13 mm. Die Verpressungsfeder 13 kann eine Länge von 64 mm aufweisen, die Verschiebungsfeder 12 kann eine Länge von 54 mm aufweisen. Die Distanz zwischen dem Boden 101 des Mittelträgers 10 und dem Einlass des Druckanschlusses 9 kann 18 mm betragen.

In der Verschlussstellung liegt die Andruckplatte 11 auf dem Außenträger 5 auf. Die Höhe des Einbauspalts 15 ist daher im Wesentlichen gleich Null. Die Verpressungsfeder 13 ist vorgespannt und kann eine Länge von 66 mm aufweisen. Dies kann ihrer maximal möglichen Länge aufgrund der Anschläge entsprechen. Die Verschiebungsfeder 12 kann eine Länge von 65 mm aufweisen. Die Distanz zwischen dem Boden des Mittelträgers 10 und dem Einlass des Druckanschlusses 9 kann 7 mm betragen. Das Volumen des Arbeitsraums 21 nimmt dabei einen minimalen Wert an. Ein zwischen der Außenseite des Bodens des Federdübels 14 und der Innenseite des Bodens 101 des Mittelträgers 10 gebildeter Freiraum kann eine Höhe von 10 mm aufweisen.

In der Kraftwechselstellung kann die Höhe des Einbauspalts 15 beispielsweise 10, 11 oder 12 mm betragen. Aufgrund der Anschläge kann die Verpressungsfeder 13 eine maximal mögliche Länge von 66 mm aufweisen. Die Verschiebungsfeder 12 kann eine Länge von 56 mm aufweisen. Die Distanz zwischen dem Boden 101 des Mittelträgers 10 und dem Einlass des Druckanschlusses 9 kann 17 mm betragen.

Alle in der vorliegenden Anmeldung angegebenen Maße, insbesondere in Millimeter angegebene, stellen exemplarische Werte dar. Die vorliegende Erfindung ist hierauf nicht beschränkt. Es wird ferner angemerkt, dass die Maße nationalen und regionalen Sicherheitsbestimmungen und Wünschen der Benutzer oder Abnehmer der vorliegenden Erfindung selbstverständlich angepasst werden können.

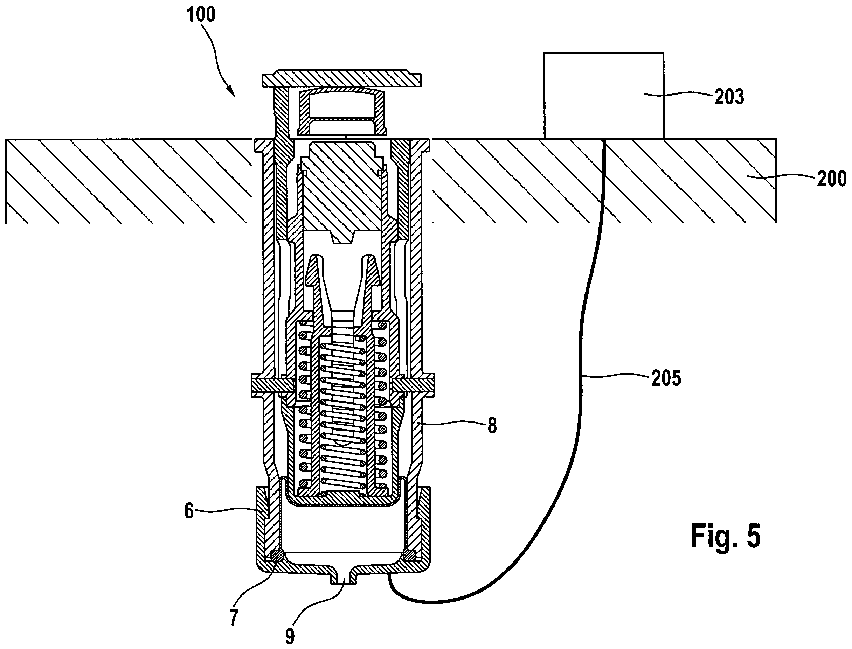

Fig. 5 zeigt als Teil-Blockbild in vereinfachter Darstellung eine Blutbehandlungsanordnung als Beispiel einer erfindungsgemäßen Anordnung 200. Die Anordnung 200 weist eine erfindungsgemäße Verbindungseinrichtung 100 mit einer Öffnungsaktorbaugruppe 6-9 auf. Sie weist ferner eine Steuereinrichtung 203 zum Einwirken auf die Öffnungsaktorbaugruppe 6-9 auf. Eine Steuerleitung 205 ist zwischen der Öffnungsaktorbaugruppe 6-9 und der Steuereinrichtung 203 vorgesehen.

Für den Fachmann ist ersichtlich, dass die in Fig. 5 gezeigte Ausführungsform mit jeder erfindungsgemäßen Verbindungseinrichtung und nicht nur mit der dort gezeigten verwirklicht werden kann. Die Darstellung der Verbindungseinrichtung 100 in Fig. 5 in ihrer konkreten Ausgestaltung dient allein dem besseren Verständnis.

Fig. 6 zeigt ein Beispiel für ein Kraft-Weg-Diagramm.

Auf der x-Achse sind verschiedene Stellungen der erfindungsgemäßen Verbindungsvorrichtung dargestellt, wie sie im Einzelnen unter Bezugnahme auf die Fig. 1 bis 4 beschrieben wurden: eine Verschlussstellung 23, eine Kraftwechselstellung 25, eine Arbeitsstellung 27 und eine Rüststellung 29.

Die Zahlenangaben in [mm] geben die jeweils zugehörige Verschiebung der Kontaktabschnitte bzw. die Spaltweite d an.

Beispielsweise beträgt ein Abstand der Kontaktabschnitte voneinander in der Verschlussstellung 23 0 mm, in der Kraftwechselstellung 25 12 mm, in der Arbeitsstellung 27 13 mm und in der Rüststellung 29 18 mm.

Auf der y-Achse ist die Kraft F in [N] angegeben. Die dicke durchgezogene Linie 30a stellt den Kraftverlauf der auf die externe Funktionseinrichtung, einen Finger, Dichtpartner usw. wirkenden Kraft dar. Sie gibt einen statistischen Zustand an.

Die gestrichelte Linie 30b und die Strich-Punkt-Linie 30c stellen Kraftverläufe des Öffnungsaktors bzw. der Öffnungsaktorbaugruppe dar. Die gestrichelte Linie 30b stellt die resultierende Kraft des Öffnungsaktors beim Öffnen dar. Die Strich-Punkt-Linie 30c stellt die resultierende Kraft des Öffnungsaktors beim Schließen dar.

Der während einer Verwendung der Verbindungsvorrichtung regelmäßig übliche Kraftverlauf ist in Fig. 6 von rechts nach links gezeigt.

Die Verbindungsvorrichtung liegt zunächst in der Rüststellung 29 vor. In der Rüststellung 29 kann die Verbindungsvorrichtung eine externe Funktionseinrichtung aufnehmen.

Beim Verfahren der Verbindungsvorrichtung aus der Rüststellung 29 in die Verschlussstellung 23 zur funktionellen Ankopplung der externen Funktionseinrichtung wird zunächst eine absolute zweite Kraft F₂ von etwa 130 N auf die Verbindungsvorrichtung aufgebracht. Beim weiteren Verfahren fällt die Kraft F₂ innerhalb eines ersten Kraftbereichs 31 mit einer ersten Steigung 33 bis zur Arbeitsstellung 27 hin ab.

Von der Arbeitsstellung 27 fällt die auf die Verbindungsvorrichtung wirkende Kraft weiter ab bis zur Kraftwechselstellung 25. Im Bereich der Kraftwechselstellung 25 tritt, wie in Fig. 6 gezeigt, eine totale Verformung von etwa 0,2 mm auf.

Die Kraft sinkt im Abschnitt 35 totaler Verformung sprungartig aus dem ersten Kraftbereich 31 von einer zweiten Kraft F₂ von etwa 125 N auf einen zweiten Kraftbereich 37 auf eine Kraft F₁ von etwa 25 N ab. Beim Übergang aus dem ersten Kraftbereich 31 in den Abschnitt 35 totaler Verformung tritt daher ein erster Knick 41 auf. Beim Übergang über den Abschnitt 35 totaler Verformung in den zweiten Kraftbereich 37 tritt ein zweiter Knick 43 auf.

Im Bereich der Kraftwechselstellung 25 und der Verschlussstellung 23 wirkt im Wesentlichen eine Kraft F₁ innerhalb des zweiten Kraftbereichs 37.

Während des Verfahrens der Verbindungsvorrichtung von der Kraftwechselstellung 25 in die Verschlussstellung 23 fällt die Kraft F₁ mit einer zweiten Steigung 39 ab.

Eine absolute Kraft F₁ in der Verschlussstellung 23 kann bei z.B. etwa 20 N liegen.

Wie aus dem Kraft-Weg-Diagramm hervorgeht, nimmt die externe Funktionseinrichtung nur entweder die Kraft F₁ oder die Kraft F₂ auf.

Die vorliegende Erfindung ist nicht auf die vorstehend beschriebenen Ausführungsformen beschränkt, diese dienen lediglich der Veranschaulichung.

## Patentansprüche

1. Verbindungsvorrichtung (100) zum Verbinden, insbesondere Ankoppeln, wenigstens einer externen Funktionseinrichtung (1) mit einer Anordnung (200), welche eine Blutbehandlungsvorrichtung ist, mittels Verpressens der externen Funktionseinrichtung (1), wobei die Verbindungsvorrichtung (100) aufweist:
- wenigstens eine Aufnahmeeinrichtung (151) mit wenigstens einem ersten Kontaktabschnitt (111) zum Aufnehmen der externen Funktionseinrichtung (1) zwischen dem ersten Kontaktabschnitt (111) und einem oder mehreren weiteren Kontaktabschnitten (A, 201), wobei der erste Kontaktabschnitt (111) mit variierbarem Abstand d zum weiteren Kontaktabschnitt (A, 201) angeordnet ist;
- wenigstens eine Verpresseinrichtung zum Verringern des variierbaren Abstandes d zum Verpressen der externen Funktionseinrichtung (1) zwischen dem ersten Kontaktabschnitt (111) und dem weiteren Kontaktabschnitt (A, 201) durch Überführen wenigstens des ersten Kontaktabschnitts (111) aus einer ersten Stellung, insbesondere Rüststellung, der Verbindungsvorrichtung (100), in welcher ein erster Abstand (d₁) zwischen dem ersten Kontaktabschnitt (111) und dem weiteren Kontaktabschnitt (A, 201) vorliegt, in eine zweite Stellung, in welcher ein zweiter Abstand (d₂) zwischen dem ersten Kontaktabschnitt (111) und dem weiteren Kontaktabschnitt (A, 201) vorliegt, wobei der zweite Abstand (d₂) geringer als der erste Abstand (d₁) ist;
wobei die Verpresseinrichtung ausgestaltet ist, um wenigstens den ersten Kontaktabschnitt (111) unter Aufbringung einer ersten Federkraft (F₁) und einer zweiten Federkraft (F₂) aus der ersten Stellung in die zweite Stellung zu überführen, wobei die zweite Federkraft (F₂) größer als die erste Federkraft (F₁) ist, **dadurch gekennzeichnet, dass** die Verpresseinrichtung wenigstens zwei Federn (12,13) zum Überführen wenigstens des ersten Kontaktabschnitts (111) aus der ersten Stellung in die zweite Stellung aufweist, wobei durch das Aufbringen der zwei unterschiedlichen Federkräfte (F₁, F₂) beim Betätigen der Verpresseinrichtung eine gestufte oder sprungweise Kraftwirkung im Kraft-Weg-Diagramm in Abhängigkeit vom Abstand der Kontaktabschnitte (111, A, 201) zueinander erzielbar ist.

2. Verbindungsvorrichtung (100) nach Anspruch 1, wobei die Verpresseinrichtung ausgestaltet ist, um wenigstens den ersten Kontaktabschnitt (111) in Abhängigkeit des Abstandes d mit der ersten Federkraft (F₁) und/oder der zweiten Federkraft (F₂) von der ersten Stellung in die zweite Stellung zu überführen.

3. Verbindungsvorrichtung (100) nach einem der vorangegangenen Ansprüche, wobei die wenigstens zwei Federn eine erste Feder und eine zweite Feder umfassen, und wobei die erste Feder ausgestaltet ist, um die erste Federkraft (F₁) aufzubringen, und die zweite Feder ausgestaltet ist, um die zweite Federkraft (F₂) aufzubringen.

4. Verbindungsvorrichtung (100) nach Anspruch 3, wobei die erste und zweite Feder derart angeordnet sind, dass die erste Feder (13) während des Übergangs von der ersten Stellung zu einer Kraftwechselstellung die erste Federkraft (F₁) ausübt und die zweite Feder während des Übergangs von der Kraftwechselstellung zur zweiten Stellung die zweite Federkraft (F₂) ausübt.

5. Verbindungsvorrichtung (100) nach Anspruch 3, wobei die erste und zweite Feder derart angeordnet sind, dass die erste Feder zusammen mit der zweiten Feder während des Übergangs von der ersten Stellung zu einer Kraftwechselstellung die erste Federkraft (F₁) ausübt und die zweite Feder während des Übergangs von der Kraftwechselstellung zur zweiten Stellung die zweite Federkraft (F₂) ausübt.

6. Verbindungsvorrichtung (100) nach einem der vorangegangenen Ansprüche, wobei die zweite Stellung eine Arbeitsstellung ist, in welcher die externe Funktionseinrichtung (1) mit der Anordnung (200) verpresst ist.

7. Verbindungsvorrichtung (100) nach einem der Ansprüche 1 bis 5, wobei die zweite Stellung eine Verschlussstellung ist, in welcher der erste Kontaktabschnitt (111) eine Abdichtung bewirkt.

8. Verbindungsvorrichtung (100) nach einem der vorangegangenen Ansprüche, welche eine pneumatisch wirkende oder pneumatisch betreibbare Öffnungsaktorbaugruppe (6-9) zum Überführen wenigstens des ersten Kontaktabschnitts (111) aus der zweiten Stellung in die erste Stellung aufweist.

9. Anordnung (200) mit wenigstens einer Verbindungsvorrichtung (100) gemäß einem der Ansprüche 1 bis 8, wobei die Anordnung eine Blutbehandlungsvorrichtung ist.

10. Anordnung (200) nach Anspruch 9, welche ferner eine Steuer- oder Regeleinrichtung (203) aufweist, welche geeignet und konfiguriert ist, um eine pneumatisch wirkende oder pneumatisch betreibbare Öffnungsaktorbaugruppe (6-9) der Verbindungsvorrichtung (100) zu steuern oder zu regeln.

11. Anordnung (200) nach einem der Ansprüche 9 oder 10, wobei die wenigstens eine externe Funktionseinrichtung (1) kraft- und/oder formschlüssig an die Anordnung (200) ankoppelbar ist zum funktionellen Ankoppeln der externen Funktionseinrichtung (1) an einen Koppelpartner (2) auf der Seite der Anordnung (200).

12. Anordnung (200) nach einem der Ansprüche 9 bis 11, wobei die Verbindungsvorrichtung (100) wenigstens ein fest an der Anordnung (200) integrierbares Trägerelement (3, 5) und/oder Stützelement (4, 17, 18, 19,20) aufweist.

13. Anordnung (200) nach einem der Ansprüche 9 bis 12, wobei wenigstens ein weiterer Kontaktabschnitt (A, 201) fest an der Anordnung (200) integrierbar oder Teil derselben ist.

## Claims

1. Connection device (100) for connecting, in particular coupling, at least one external functional means (1) to an arrangement (200) being a blood treatment apparatus by pressing the external functional means (1), wherein the connection device (100) comprises:
- at least one reception means (151) having at least one first contact portion (111) for receiving the external functional means (1) between the first contact portion (111) and one or several further contact portions (A, 201), wherein the first contact portion (111) is arranged at a variable spacing d from the further contact portion (A, 201);
- at least one pressing means for reducing the variable spacing d for pressing the external functional means (1) between the first contact portion (111) and the further contact portion (A, 201) by transferring at least the first contact portion (111) from a first position, in particular a set-up position, of the connection device (100), in which a first spacing (d1) between the first contact portion (111) and the further contact portion (A, 201) is present, into a second position in which a second spacing (d2) between the first contact portion (111) and the further contact portion (A, 201) is present, wherein the second spacing (d2) is smaller than the first spacing (d1);
- wherein the pressing means is configured for transferring at least the first contact portion (111) from the first position into the second position by applying a first spring force (F1) and a second spring force (F2), wherein said second spring force (F2) is larger than the first spring force (F1);
**characterized in that**:
- the pressing means comprises at least two spring elements (12, 13) for transferring at least the first contact portion (111) from the first position into the second position, wherein a stepped or abrupt change of force application in the force-path-diagram as a function of of the mutual spacing of the contact portions (111, A, 201) is achievable by applying the two different spring forces (F1, F2) during operation of the pressing means.

2. Connection device (100) according to claim 1, wherein the pressing means is configured for transferring at least the first contact portion (111) from the first position into the second position depending on the spacing d by means of the first spring force (F1) and/or of the second spring force (F2).

3. Connection device (100) according to any one of the preceding claims, wherein the at least two springs comprise a first spring and a second spring and wherein the first spring is configured for applying the first spring force (F1) and the second spring is configured for applying the second spring force (F2) .

4. Connection device (100) according to claim 3, wherein the first and the second spring are arranged such that the first spring (13) exerts the first spring force (F1) during the transition from the first position to a force change position, and the second spring exerts the second spring force (F2) during the transition from the force change position to the second position.

5. Connection device (100) according to claim 3, wherein the first and the second spring are arranged such that the first spring together with the second spring exert the first spring force (F1) during the transition from the first position to a force change position, and the second spring exerts the second spring force (F2) during the transition from the force change position to the second position.

6. Connection device (100) according to any one of the preceding claims, wherein the second position is a working position in which the external functional means (1) is pressed with the arrangement (200).

7. Connection device (100) according to any one of the claims 1 to 5, wherein the second position is a closure position in which the first contact portion (111) effects sealing.

8. Connection device (100) according to any one of the preceding claims, which comprises a pneumatically acting or pneumatically operable opening actor subassembly (6-9) for transferring at least the first contact portion (111) from the second position into the first position.

9. Arrangement (200) comprising at least one connection device (100) according to any one of claims 1 to 8, wherein the arrangement is a blood treatment apparatus.

10. Arrangement (200) according to claim 9 comprising further a control or regulating device (203) which is suitable and configured for controlling or regulating a pneumatically acting or pneumatically operable opening actor subassembly (6-9) of the connection device (100).

11. Arrangement (200) according to any one of the claims 9 or 10, wherein the at least one external functional means (1) is adapted to be coupled by frictional and/or form closure connection to the arrangement (200), in order to functionally couple the external functional means (1) to a coupling partner (2) on the side of the arrangement (200).

12. Arrangement (200) according to any one of the claims 9 to 11, wherein the connection device (100) comprises at least one carrier member (3, 5) and/or support member (4, 17, 18, 19, 20) adapted to be fixedly integrated on the arrangement (200).

13. Arrangement (200) according to any one of the claims 9 to 12, wherein at least one further contact portion (A, 201) is adapted to be fixedly integrated on the arrangement (200) or is a part thereof.

## Revendications

1. Dispositif de connexion (100) pour connecter, notamment coupler, au moins un moyen fonctionnel externe (1) à un (arrangement (200) étant un appareil de traitement du sang par pression du moyen fonctionnel externe (1), ledit dispositif de connexion (100) comprenant:
- au moins un moyen de réception (151) ayant au moins une première partie de contact (111) pour recevoir le moyen fonctionnel externe (1) entre la première partie de contact (111) et une ou plusieurs autres parties de contact (A, 201), la première partie de contact (111) étant disposée à une distance variable d de l'autre partie de contact (A, 201);
- au moins un moyen de pression pour réduire la distance variable d prévu pour presser le moyen fonctionnel externe (1) entre la première partie de contact (111) et l'autre partie de contact (A, 201) en transférant au moins la première partie de contact (111) à partir d'une première position du dispositif de connexion (100), en particulier une position d'équipement, dans laquelle une première distance (d1) est présente entre la première partie de contact (111) et l'autre partie de contact (A, 201), vers une seconde position dans laquelle une seconde distance (d2) est présente entre la première partie de contact (111) et l'autre partie de contact (A, 201), ladite seconde distance (d2) étant plus petite que la première distance (d1);
- où le moyen de pression est configuré pour transférer au moins la première partie de contact (111) de la première position vers la seconde position par application d'une première force de ressort (F1) et d'une seconde force de ressort (F2), et où la seconde force de ressort (F2) est plus grande que la première force de ressort (Fl);
**caractérisé en ce que**
- le moyen de pression comprend au moins deux ressorts (12, 13) pour transférer au moins la première partie de contact (111) de la première position vers la seconde position, et où une action de force étagée ou brusquement changeante dans le diagramme force-trajectoire peut être réalisée en fonction de l'espacement mutuel des parties de contact (111, A, 201) grâce à l'application des deux différentes forces de ressort (F1, F2) lors de l'actionnement du moyen de pression.

2. Dispositif de connexion (100) selon la première revendication, où le moyen de pression est conçu pour au moins transférer la première partie de contact (111) de la première position vers la seconde position en fonction de la distance d et au moyen de la première force de ressort (F1) et/ou de la seconde force de ressort (F2).

3. Dispositif de connexion (100) selon l'une quelconque des revendications précédentes, où au moins les deux ressorts comprennent un premier ressort et un second ressort, et où le premier ressort est conçu pour établir la première force de ressort (F1) et le second ressort est conçu pour établir la seconde force de ressort (F2).

4. Dispositif de connexion (100) selon la revendication 3, où le premier ressort et le second ressort sont agencés de sorte que le premier ressort (13) exerce la première force de ressort (F1) lors du passage de la première position vers une position de transition de force et que le second ressort exerce la seconde force de ressort (F2) lors du passage de la position de transition de force vers la seconde position.

5. Dispositif de connexion (100) selon la revendication 3, où le premier ressort et le second ressort sont agencés de sorte que le premier ressort en collaboration avec le second ressort exerce la première force de ressort (F1) lors du passage de la première position vers une position de transition de force et que le second ressort exerce la seconde force de ressort (F2) lors du passage de la position de transition de force vers la seconde position.

6. Dispositif de connexion (100) selon l'une quelconque des revendications précédentes, où la seconde position est une position de fonctionnement dans laquelle le moyen fonctionnel externe (1) est pressé avec l'arrangement (200).

7. Dispositif de connexion (100) selon l'une quelconque des revendications 1 à 5, où la seconde position est une position de fermeture dans laquelle la première partie de contact (111) provoque une étanchéification.

8. Dispositif de connexion (100) selon l'une quelconque des revendications précédentes comprenant un sous-ensemble d'acteurs d'ouverture (6-9) à action pneumatique ou actionnable de façon pneumatique prévu pour transférer au moins la première partie de contact (111) de la seconde position vers la première position.

9. Arrangement (200) comprenant au moins un dispositif de connexion (100) selon l'une quelconque des revendications 1 à 8, où l'arrangement est un appareil de traitement du sang.

10. Arrangement (200) selon la revendication 9 comprenant de plus un dispositif de contrôle ou de réglage (203) adapté et conçu pour contrôler ou régler un sous-ensemble d'acteurs d'ouverture (6-9) à action pneumatique ou actionnable de façon pneumatique du dispositif de connexion (100).

11. Arrangement (200) selon l'une quelconque des revendications 9 ou 10, où au moins le moyen fonctionnel externe (1) est adapté pour être couplé à l'arrangement (200) par complémentarité de force et/ou de forme de façon à ce que le moyen fonctionnel externe (1) soit couplé de façon fonctionnelle avec un partenaire de couplage (2) sur un coté de l'arrangement (200).

12. Arrangement (200) selon l'une quelconque des revendications 9 à 11, où le dispositif de connexion (100) comprend au moins un élément de support (3, 5) et/ou un élément de soutien (4, 17, 18, 19, 20) pouvant être intégré solidement sur l'arrangement (200).

13. Arrangement (200) selon l'une quelconque des revendications 9 à 12, où au moins une autre partie de contact (A, 201) peut être intégrée solidement sur l'arrangement (200) ou forme partie de celui-ci.
